# FASCICULE DE BREVET EUROPEEN

(11) **EP 3 559 617 B1**
(45) Date de publication et mention de la délivrance du brevet: **07.07.2021**
(21) Numéro de dépôt: 17808428.1
(22) Date de dépôt: 30.11.2017
(51) Int. Cl.: G01J 3/02, G01J 3/10, G01J 5/08, G01J 5/12, G01J 3/44, G01N 21/65, G01N 33/22

(54) **METHODE OPTIMISEE DE DETECTION DE LA FORMATION D'HYDRATES DE GAZ**
OPTIMIERTES VERFAHREN ZUR ERKENNUNG DER BILDUNG VON GASHYDRATEN
OPTIMISED METHOD FOR DETECTING THE FORMATION OF GAS HYDRATES

(30) Priorité: 21.12.2016 FR 1662982
(43) Date de publication de la demande: 30.10.2019
(73) Titulaire: IFP Energies nouvelles, 92852 Rueil-Malmaison (FR)
(72) Inventeur: DELPOUX, Olivier, 38500 Voiron (FR); FROT, Didier, 78100 Saint Germain en Laye (FR); SINQUIN, Anne, 95870 Bezons (FR); SAGNARD, Corinne, 01150 Blyes (FR); LACHET, Véronique, 91400 Orsay (FR)
(74) Mandataire: IFP Energies nouvelles
(86) Numéro de dépôt international: PCT/EP2017/080968
(87) Numéro de publication internationale: WO 2018/114267

(56) Documents cités:
- WO-A1-2010/043824
- FR-A1- 2 984 504
- SCHICKS J M ET AL: "Raman spectra of gas hydrates-differences and analogies to ice 1h and (gas saturated) water", SPECTROCHIMICA ACTA. PART A: MOLECULAR AND BIOMOLECULAR SPECTROSCOPY, ELSEVIER, AMSTERDAM, NL, vol. 61, no. 10, 1 août 2005 (2005-08-01), pages 2399-2403, XP027703263, ISSN: 1386-1425 [extrait le 2005-08-01]
- CHI LO ET AL: "Investigations of surfactant effects on gas hydrate formation via infrared spectroscopy", JOURNAL OF COLLOID AND INTERFACE SCIENCE, ACADEMIC PRESS,INC, US, vol. 376, no. 1, 3 mars 2012 (2012-03-03), pages 173-176, XP028410237, ISSN: 0021-9797, DOI: 10.1016/J.JCIS.2012.03.012 [extrait le 2012-03-12]
- XU XUE ET AL: "Detection of water-ice phase transition based on Raman spectrum", JOURNAL OF RAMAN SPECTROSCOPY, vol. 44, no. 7, 24 juillet 2013 (2013-07-24), pages 1045-1048, XP055367175, GB ISSN: 0377-0486, DOI: 10.1002/jrs.4310
- ANDREAS BRAEUER ET AL: "A Raman technique applicable for the analysis of the working principle of promoters and inhibitors of gas hydrate formation", JOURNAL OF RAMAN SPECTROSCOPY, vol. 46, no. 11, 25 novembre 2015 (2015-11-25), pages 1145-1149, XP055366542, GB ISSN: 0377-0486, DOI: 10.1002/jrs.4744

## Description

La présente invention concerne le domaine technique de la production et du stockage de gaz naturel, et plus généralement les fluides essentiellement gazeux susceptibles de former des cristaux d'hydrates ou de clathrates dans une conduite.

L'invention concerne une méthode optimisée permettant de détecter la présence ou la propension à la formation d'hydrates d'un gaz ou d'hydrates d'un mélange de gaz, au sein d'un fluide essentiellement gazeux.

On connaît des cellules pour étudier la capacité d'un système formé de liquide et de gaz à former des hydrates de gaz. Dans des installations laboratoire, installations pilotes et/ou industrielles, la formation des hydrates de gaz est détectée soit par une augmentation de la température car la cristallisation est exothermique, soit, lorsque le dispositif de travail est respectivement fermé ou semi-fermé (permettant le maintien de la pression) par une chute de pression ou par une brusque consommation de gaz. Il est également possible de détecter la formation d'hydrates par examen visuel. Il faut souligner que dans la plupart de ces méthodes, il est nécessaire de former (ou de dissocier) un grand nombre de cristaux d'hydrates pour obtenir un signal significatif. Dans le cas de systèmes gazeux comprenant des faibles teneurs en eau, des cellules d'équilibre avec mesures de la teneur en eau par chromatographie gazeuse ou par coulométrie sont employées.

Les hydrates de gaz sont des cristaux composés d'un réseau de molécules d'eau stabilisé par des formateurs d'hydrates (tels que du CO₂, de l'H₂S, de l'azote...). Les hydrates de gaz se forment dans des conditions de hautes pressions et à basses températures. Si ces cristaux se forment, ils croissent, s'agglomèrent et conduisent à un bouchage des canalisations. La remédiation de tels bouchages est longue, difficile et dangereuse. Actuellement, les opérateurs mettent en œuvre des solutions techniques lourdes et coûteuses pour éviter la formation de ces cristaux.

Un des objectifs de la présente invention est de fournir une méthode de mesure et de détection précoce de la formation d'hydrates de gaz, permettant la mise en œuvre de techniques de remédiation efficaces vis-à-vis de la formation d'hydrates.

La spectrométrie Raman est une technique non destructive et non invasive d'étude des vibrations de liaisons de molécules qui est, à l'heure actuelle, communément utilisée pour investiguer la structure et la composition d'hydrates de gaz naturel ou synthétique. En effet, il est connu que, dans le cas d'hydrates de gaz pur, la spectrométrie Raman permet d'identifier au travers des modes de vibration des molécules hôtes, la structure de l'hydrate de gaz (de type SI, SII ou SH) et de déterminer quantitativement les occupations relatives des différents types de cavités de ces différents cristaux d'hydrates. Dans le cas d'hydrates mixtes (stabilisés par un mélange de gaz), la technique permet d'identifier qualitativement la structure de l'hydrate formé et la nature des molécules hôtes.

La spectrométrie Raman a déjà été utilisée comme un moyen d'étudier la formation d'eau solide.

On connait les documents suivants :
- WO 2010/043824 A1, qui concerne un procédé de détermination de la phase solide/liquide d'une solution aqueuse ;
- Schicks J. M., Erzinger, J., Ziemann, M. A., "Raman spectra of gas hydrates-differences and analogies to ice Ih and (gas saturated) waters, SPECTROCHIMICA ACTA. PART A: MOLECULAR AND BIOMOLECULAR SPECTROSCOPY, ELSEVIER, AMSTERDAM, NL, vol. 61, no. 10, 1 août 2005 (2005-08-01), pages 2399-2403, XP027703263, ISSN: 1386-1425, qui concerne les similarités et les dissemblances entre le spectre de Raman de la molécule hôte et le spectre de Raman d'hydrates de gaz ;
- FR 2984504 A1, qui concerne un dispositif et une méthode de mesure et de détection de la présence d'hydrates de gaz.

On connait en outre les documents suivants qui illustrent le contexte technique :
- Lo, Chi & Zhang, Junshe & Somasundaran, Ponisseril & Lee, Jae, "Investigations of surfactant effects on gas hydrate formation via infrared spectroscopy", JOURNAL OF COLLOID AND INTERFACE SCIENCE, ACADEMIC PRESS,INC, US, vol. 376, no. 1, 3 mars 2012 (2012-03-03), pages 173-176, XP028410237, ISSN: 0021-9797, D01: 10.1016/J.JCIS.2012.03.012 ;
- Xue, Xu & He, Zhi & Liu, Jing, "Détection of water-ice phase transition based on Raman spectrum", JOURNAL OF RAMAN SPECTROSCOPY, vol. 44, no. 7, 24 juillet 2013 (2013-07-24), pages 1045-1048, XP055367175, GB ISSN: 0377-0486, D01: 10.1002/jrs.4310 ;
- Braeuer, Andreas & Hankel, Robert & Mehnert, Markus & Schuster, Julian & Will, Stefan, "A Raman technique applicable for the analysis of the working principle of promoters and inhibitors of gas hydrate formation", JOURNAL OF RAMAN SPECTROSCOPY, vol. 46, no. 11, 25 novembre 2015 (2015-11-25), pages 1145-1149, XP055366542, GB ISSN: 0377-0486, D01: 10.1002/jrs.4744.

La présente invention est fondée sur l'utilisation de spectres Raman dans la zone des modes de vibration des liaisons OH d'un milieu contenant de l'eau susceptible de former des cristaux solides (tels que de la glace et/ou des hydrates), avec la combinaison d'une mesure de la température. En limitant l'utilisation de spectres Raman dans la zone des modes de vibration des liaisons OH, la méthode de détection des hydrates devient moins longue et moins coûteuse que les méthodes actuelles : en effet, il n'est pas nécessaire de balayer l'ensemble du spectre.

Ainsi, l'objet de la présente invention concerne une méthode de détection de la présence d'hydrates de gaz et/ou de glace dans un milieu contenant de l'eau et susceptible de former des cristaux solides, caractérisée en ce qu'elle comporte au moins les étapes suivantes :
- on mesure en au moins un point de mesure dans ledit milieuau moins deux valeurs caractéristiques des spectres Raman correspondants à deux modes de vibration distincts des liaisons OH de l'eau, et on détermine le rapport τ desdites deux valeurs caractéristiques ;
- on détermine la température T dans ledit milieu audit point de mesure desdits spectres ;
- on compare le rapport τ à une valeur **τ**₀ correspondant à un seuil prédéterminé de formation desdits cristaux pour ladite température T ; et
- on détermine une présence d'eau sous forme solide à partir de ladite comparaison et on distingue entre une présence de glace ou une présence d'hydrates de gaz à partir d'une comparaison entre ladite température mesurée T avec la température de la formation de la glace dans les conditions de mesure.

Les deux modes de vibration correspondent à un nombre d'onde situé à 3160 cm⁻¹ ± 40 cm⁻¹, et à un nombre d'onde situé à 3400 ±150 cm⁻¹.

La valeur caractéristique peut correspondre à l'intensité desdits deux modes sur les spectres, ou à une valeur directement liée à l'intensité, par exemple l'intégrale dudit spectre centré sur lesdits modes de vibration.

Selon un mode de réalisation de l'invention, on peut régler la température à proximité du point de mesure desdites deux valeurs caractéristiques pour anticiper la formation de cristaux solides, par exemple d'hydrates.

Avantageusement, on peut déduire la présence de cristaux d'hydrates si le rapport **τ** est supérieur à une valeur d'étalonnage **τ**₀ et si si la température mesurée est supérieure à la température de formation de la glace Tf dans les conditions de mesure.

Selon un mode de réalisation de l'invention, en cas de présence d'hydrates, on injecte un additif anti-hydrates dans ledit milieu.

La présente invention sera mieux comprise et ses avantages apparaîtront plus clairement à la lecture de la description qui suit d'exemples de mise en œuvre, nullement limitatifs, et illustrés par les figures ci-après annexées, parmi lesquelles :
- La figure 1 montre des spectres Raman en fonction de la température du milieu considéré ;
- La figure 2 montre l'évolution du rapport **T** de l'intensité Raman de deux modes différents de vibrations des liaisons OH.

La présente invention concerne une méthode de détection de l'apparition de cristaux d'hydrates, et plus généralement de cristaux solides dans un milieu comprenant de l'eau, par le couplage de valeurs caractéristiques obtenues à partir d'une spectrométrie Raman dans la gamme spectrale des modes de vibration des liaisons OH, et d'une sonde de température. On appelle cristaux solides des cristaux d'hydrates de gaz et/ou des cristaux de glace. La détection des modes de vibration des liaisons OH permet de qualifier les liaisons OH présentes dans l'eau liquide, dans la glace, et/ou dans les hydrates. De cette manière, il est possible de déterminer si de la glace et/ou des hydrates se sont formés.

On rappelle que la spectrométrie Raman est une méthode optique d'observation et de caractérisation de la composition moléculaire et de la structure externe d'un matériau.

La spectrométrie Raman exploite le phénomène physique selon lequel un milieu modifie légèrement la fréquence de la lumière y circulant. La spectroscopie Raman consiste à envoyer une lumière monochromatique sur l'échantillon et à analyser la lumière diffusée. Les informations obtenues par la mesure et l'analyse de ce décalage permettent de remonter à certaines propriétés du milieu, par la spectroscopie.

Le signal issu du spectromètre Raman est transmis au milieu par une sonde, appelée sonde Raman. La sonde Raman permet également de conduire le signal du point de mesure vers le spectromètre. Avantageusement, la sonde Raman peut être une sonde en immersion dans le milieu comprenant de l'eau. La sonde Raman, en immersion dans le milieu comprenant de l'eau, peut être sous forme d'un tube cylindrique en acier connecté à deux fibres optiques, la fibre « aller » (ou première fibre) qui conduit le signal issu de la source laser vers le point de mesure, et la fibre « retour » (ou seconde fibre) qui conduit le signal Raman du point de mesure vers le spectromètre.

L'extrémité immergée de la sonde est constituée d'une fenêtre, généralement en saphir, laissant passer les rayonnements lumineux.

Cette extrémité est plongée directement dans le milieu à analyser, permettant une analyse *in situ.* La, ou les sonde(s) en immersion peuvent être placée(s) en différents points de l'unité en fonction de l'objectif poursuivi.

Selon un mode de réalisation de l'invention, le spectromètre Raman utilisé peut être un spectromètre Raman dispersif équipé d'un laser ayant une longueur d'onde d'excitation inférieure à 785 nm (par exemple un Nd-YAG doublé en fréquence (λ=532 nm)), d'un miroir d'entrée torique (améliorant la qualité de l'image sur le détecteur par la correction des aberrations optiques, en particulier de l'astigmatisme) et d'un détecteur de type CCD. Le choix du laser et du détecteur sont conditionnés par la recherche de l'optimum en termes de rapport signal sur bruit dans la gamme spectrale des modes de vibration des liaisons OH.

A proximité du point de l'unité où le spectre Raman est mesuré, une sonde de température (par exemple thermocouple, ou au moyen d'une troisième fibre optique qui permet de déporter le capteur ou tout autre moyen de mesure de la température) peut être installée de manière à disposer simultanément du spectre Raman et de la température de la zone échantillon. Ainsi, chaque point de mesure de la spectroscopie Raman est associé à une mesure de température située au voisinage du point de mesure, permettant de mesurer la température du fluide au moins dans le voisinage de ce point de mesure.

Alternativement, la température peut être connue par tout autre moyen, par exemple, mesure en un autre point, conditions imposées au milieu, etc..

Les deux données (spectre Raman et température) peuvent être envoyées vers des moyens d'analyse, notamment des moyens informatiques (par exemple un PC) contrôlant la chaîne analytique, en vue d'une exploitation de ces mesures.

Une méthode mathématique de décomposition spectrale est ensuite utilisée afin d'évaluer, après une soustraction de la ligne de base (méthode bien connue de l'homme de l'art), une valeur caractéristique pour chacun des deux modes de vibration des liaisons OH (appelés également modes de vibration de l'eau) suivants :
- un premier mode de vibration de l'eau (désigné ci-après mode A), tel que celui avec un nombre d'onde situé à 3160 cm⁻¹ +/- 40 cm⁻¹.
- et un second mode de vibration de l'eau (désigné ci-après mode B), tel que celui avec un nombre d'onde situé à 3400 +/-150 cm⁻¹.

En limitant l'utilisation de spectres Raman dans la zone des modes de vibration des liaisons OH, la méthode de détection des hydrates devient moins longue et moins coûteuse que les méthodes actuelles : en effet, il n'est alors pas nécessaire de balayer l'ensemble du spectre.

Par « valeur caractéristique », on entend l'intensité du signal, ou une valeur directement liée à l'intensité, par exemple l'aire (obtenue par intégration du spectre sur des bandes correspondant aux deux modes de vibration de l'eau).

La position des bandes correspondant aux modes de vibration A et B peut être donnée en nombre d'onde (cm⁻¹) ou en longueur d'onde (nm). On rappelle que le nombre d'onde est une grandeur égale à l'inverse de la longueur d'onde. Cette position des bandes est toujours donnée en relatif (shift Raman) par rapport à la position du laser incident (la position des bandes exprimée en longueur d'onde dépend de la longueur d'onde du laser incident du spectroscope Raman).

Une fois que les deux valeurs caractéristiques sont déterminées, on calcule un rapport τ de ces deux valeurs caractéristiques. De préférence, le rapport correspond au rapport du premier mode de vibration de l'eau (mode A) par le deuxième mode de vibration de l'eau (mode B).

Le rapport τ est ensuite comparé à des valeurs limites τ₀ déterminées préalablement par étalonnage dans le milieu considéré. Les valeurs limites **τ**₀ peuvent dépendre du milieu, de la température, de la pression, etc. Le rapport **τ**₀ peut dépendre de la température, d'où l'intérêt de recourir à une mesure de température couplée à la mesure Raman. Si **τ** > **τ₀**, alors le système contient de l'eau sous forme solide (hydrates et/ou glace). Si **τ** < **τ₀**, alors le système ne contient pas d'eau sous forme solide (hydrates et/ou glace). De plus, dans le cas où **τ** > **τ₀**, si la température T mesurée à proximité dudit point de mesure est supérieure à la température Tf de formation de la glace dans les conditions de mesure, on peut distinguer entre une présence de glace ou une présence d'hydrates de gaz : si **τ** > **τ**₀ et T > Tf, alors on peut mettre en évidence la présence d'hydrates de gaz.

La température Tf dépend notamment du milieu comprenant de l'eau et de la pression. En particulier, la température Tf peut être plus élevée en présence d'additif.

Selon un exemple de réalisation de l'invention, le rapport **τ**₀ peut est compris entre 1 et 1,4 pour la détection de la formation d'hydrates dans un milieu comprenant du méthane.

L'opération d'étalonnage est possiblement réalisée à différentes températures, dans des conditions représentatives d'opérations industrielles du milieu comprenant de l'eau.

En résumé, à partir de la procédure d'étalonnage, de la mesure en ligne du spectre Raman et de la température T au voisinage du point de mesure, on détermine une valeur limite permettant de statuer sur la formation ou non d'eau sous forme solide, notamment sous forme d'hydrates de gaz.

Selon une mise en œuvre de l'invention, un dispositif de refroidissement du milieu au niveau du point de mesure peut être adjoint de façon à pouvoir contrôler la température du milieu (en imposant une gamme de températures au niveau du point de mesure) afin d'anticiper la formation d'hydrates, ou plus généralement d'eau sous forme solide.

Selon un mode de réalisation de l'invention, si on détecte une formation d'hydrates et/ou de glace au niveau du point de mesure après refroidissement du milieu au niveau du point de mesure, il est possible d'éviter la formation d'hydrates au sein du milieu, en injectant un additif anti-hydrate dans le milieu comprenant de l'eau. Ainsi, il est possible d'anticiper la prévention d'hydrates dans le milieu comprenant de l'eau.

La méthode peut comprendre les étapes suivantes :
- on envoie en au moins un point du milieu un signal lumineux dont la longueur d'onde est inférieure à 785 nm,
- on récupère le spectre Raman au point considéré,
- on traite le spectre Raman selon la méthode décrite ci-dessus (en mesurant les valeurs caractéristiques pour les deux modes de vibration des liaisons OH),
- on récupère à l'issue de ce traitement la valeur du rapport d'intensité **τ**,
- on mesure la température T au voisinage du point de mesure,
- on compare la valeur du rapport **τ** avec une valeur de référence **τ**₀,
- en fonction de l'écart entre la valeur mesurée **τ** et la valeur de référence **τ₀**, et en fonction de la température mesurée, on statue sur la présence ou non de cristaux solides de glace ou d'hydrates.

En fonction de cette information, on peut agir sur au moins une variable d'action, par exemple la température, la pression, l'injection d'additif, ou le débit du fluide, pour éviter la formation d'hydrates (ou de glace) dans le milieu comprenant de l'eau.

Dans une variante, la température T au voisinage du point de mesure est contrôlée. On peut ajouter une étape préliminaire qui vise à refroidir ledit point de mesure. La méthode permet alors d'anticiper une température de formation d'hydrates en conditions réelles.

### Exemple

Les caractéristiques et avantages de la méthode selon l'invention apparaîtront plus clairement à la lecture de l'exemple d'application ci-après. Dans cet exemple, le milieu est composé de méthane en phase gaz à une pression de 70 bars et d'une faible quantité d'eau dans une enceinte dans laquelle est placée une sonde de température et une sonde Raman ¼". Le spectromètre utilisé est un RXN2C de la société Kaiser avec une longueur d'excitation de 532 nm.

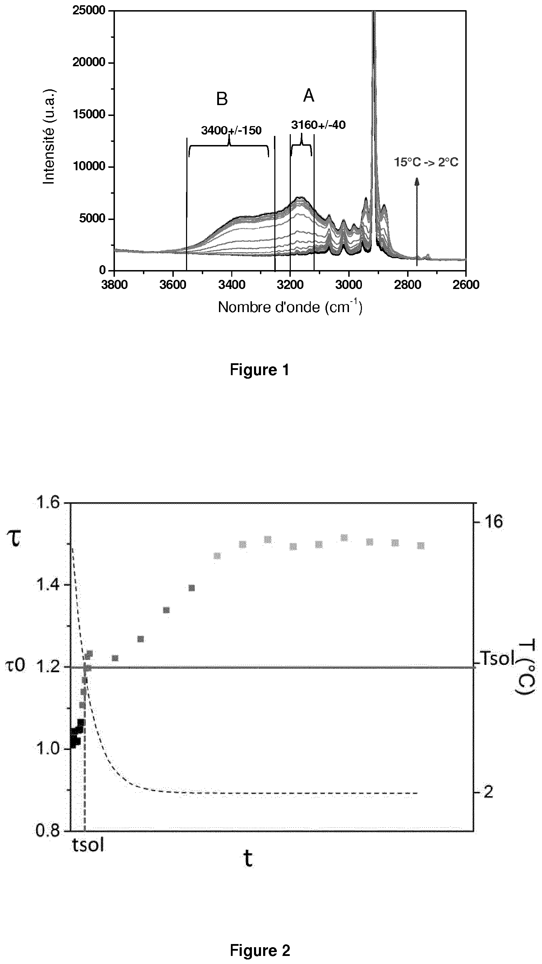

Les spectres Raman illustrés sur la figure 1 ont été enregistrés lors du refroidissement de l'enceinte entre 15°C et 2°C. Chaque courbe représentée correspond à une température de l'enceinte.

On peut voir sur cette figure que la composante majoritaire est celle du méthane en phase gaz, avec un pic principal situé vers 2917 cm⁻¹ correspondant à la vibration d'élongation symétrique des liaisons CH du méthane. Dans la méthode proposée, on ne cherche pas exploiter les modes de vibration de ces liaisons CH ; mais on se base sur l'analyse des modes de vibration des liaisons OH de l'eau que l'on peut voir figure 1 dans la zone comprise entre 3100 cm⁻¹ et 3600 cm⁻¹ environ. Dans cette zone, on peut voir deux modes de vibration de l'eau : un premier mode (désigné par la lettre A sur la figure 1) situé vers 3160 cm⁻¹ et un second mode (désigné par la lettre B) situé vers 3400 cm⁻¹. On observe que le domaine de vibration des liaisons OH subit des modifications lors de la descente en température. Plus précisément, on peut noter que les intensités relatives des deux modes de vibration de l'eau évoluent au fur et à mesure de la baisse de température, avec une augmentation plus importante de l'intensité du mode A. Cette évolution est attribuée à la formation d'eau solide sous forme d'hydrates lors de la baisse de température de 15°C à 2°C (car les températures sont supérieures à la température de fusion de la glace).

Dans cet exemple, on mesure les intensités aux nombres d'onde de 3173 cm⁻¹ et 3413 cm⁻¹. On calcule ensuite le rapport τ des deux intensités (I(3173)/I(3413)) en fonction du temps (Figure 2) ou indifféremment en fonction de la température puisque la température est abaissée au cours du temps dans cet essai. Sur la figure 2, le rapport des intensités **τ** est représenté par des carrés gris et la variation de la température T en °C est illustrée par la courbe en pointillée. La comparaison des valeurs du rapport **τ**, comprises ici entre environ 1 et 1,5, à une valeur **τ**₀ de référence fixée ici à 1,2 suite à un étalonnage préalable, permet de statuer sur la présence d'eau sous forme solide. Dans la région **τ** < **τ₀**, le système ne contient pas d'eau sous forme solide. Dans la région **τ** > **τ₀**, le système contient de l'eau sous forme solide. On note tₛₒₗ le temps correspondant au passage d'une région à une autre, c'est-à-dire correspondant au moment où des cristaux solides se forment. La mesure de la température T à proximité du point de mesure des spectres Raman permet de convertir ce temps tₛₒₗ en une température Tₛₒₗ d'apparition des particules solides. Dans cet exemple, la température varie entre 15°C et 2°C, la température Tₛₒₗ d'apparition des particules solides est supérieure à la température de formation de la glace, ce qui permet de plus de conclure sur l'apparition de cristaux de type hydrates de gaz, plutôt que de type glace.

## Revendications

1. Méthode de détection de la présence d'hydrates de gaz ou de glace dans un milieu contenant de l'eau et susceptible de former des cristaux solides, **caractérisée en ce qu'**elle comporte au moins les étapes suivantes :
- on mesure en au moins un point de mesure dans ledit milieu deux valeurs caractéristiques des spectres Raman correspondants à deux modes de vibration distincts des liaisons OH de l'eau, lesdits deux modes de vibration correspondant à un premier nombre d'onde situé à 3160 cm⁻¹ ± 40 cm⁻¹, et à un deuxième nombre d'onde situé à 3400 cm⁻¹ ±150 cm⁻¹, et on détermine le rapport **τ** desdites deux valeurs caractéristiques ;
- on détermine la température T dans ledit milieu audit point de mesure desdits spectres ;
- on compare le rapport **τ** à une valeur **τ**₀ correspondant à un seuil prédéterminé de formation desdits cristaux pour ladite température T ; et
- on détermine une présence d'eau sous forme solide à partir de ladite comparaison et on distingue entre une présence de glace ou une présence d'hydrates de gaz à partir d'une comparaison entre ladite température mesurée T avec la température de la formation de la glace dans les conditions de mesure.

2. Méthode selon la revendication 1, dans laquelle ladite valeur caractéristique correspond à l'intensité dudit spectre pour lesdits deux modes de vibration, ou à une valeur directement liée à l'intensité, par exemple l'intégrale dudit spectre centré sur lesdits modes de vibration.

3. Méthode selon l'une des revendications précédentes, dans laquelle on fait varier ladite température au voisinage dudit point de mesure desdites deux valeurs caractéristiques pour anticiper la formation de cristaux solides, par exemple d'hydrates.

4. Méthode selon l'une des revendications précédentes, dans laquelle on déduit la présence de cristaux d'hydrates si le rapport **τ** est supérieur à une valeur d'étalonnage **τ**₀ et si la température mesurée est supérieure à la température de formation de la glace Tf dans les conditions de mesure.

5. Méthode selon l'une des revendications précédentes, dans laquelle, en cas de présence d'hydrates, on injecte un additif anti-hydrates dans ledit milieu.

## Patentansprüche

1. Verfahren zum Nachweis des Vorhandenseins von Gashydraten oder von Eis in einem Medium, das Wasser enthält und zur Bildung von festen Kristallen in der Lage ist, **dadurch gekennzeichnet, dass** es mindestens die folgenden Schritte umfasst:
- Messen von zwei charakteristischen Werten der Ramanspektren, die zwei bestimmten Schwingungsmoden der OH-Bindungen von Wasser entsprechen, an mindestens einem Messpunkt in dem Medium, wobei die beiden Schwingungsmoden einer ersten bei 3160 cm⁻¹ ± 40 cm⁻¹ gelegenen Wellenzahl und einer zweiten bei 3400 cm⁻¹ ± 150 cm⁻¹ gelegenen Wellenzahl entsprechen, und Bestimmen des Verhältnisses τ der beiden charakteristischen Werte;
- Bestimmen der Temperatur T in dem Medium an dem Punkt der Messung der Spektren;
- Vergleichen des Verhältnisses τ mit einem Wert τ₀, der einem zuvor festgelegten Grenzwert der Bildung der Kristalle bei der Temperatur T entspricht; und,
- ausgehend von dem Vergleich, Bestimmen des Vorhandenseins von Wasser in fester Form und, ausgehend von einem Vergleich zwischen der gemessenen Temperatur T und der Eisbildungstemperatur unter den Messbedingungen, Unterscheiden zwischen dem Vorhandensein von Eis oder dem Vorhandensein von Gashydraten.

2. Verfahren nach Anspruch 1, bei dem der charakteristische Wert der Intensität des Spektrums für die beiden Schwingungsmoden oder einem direkt mit der Intensität in Beziehung stehenden Wert, beispielsweise dem auf die Schwingungsmoden zentrierten Integral des Spektrums, entspricht.

3. Verfahren nach einem der vorhergehenden Ansprüche, bei dem man die Temperatur in der Umgebung des Punkts der Messung der beiden charakteristischen Werte zur Antizipierung der Bildung von festen Kristallen, beispielsweise von Hydraten, verändert.

4. Verfahren nach einem der vorhergehenden Ansprüche, bei dem man dann, wenn das Verhältnis τ über einem Kalibrierwert τ₀ liegt und wenn die gemessene Temperatur über der Eisbildungstemperatur Tf unter den Messbedingungen liegt, das Vorhandensein von Hydratkristallen ableitet.

5. Verfahren nach einem der vorhergehenden Ansprüche, bei dem man beim Vorhandensein von Hydraten ein Hydratinhibierungsadditiv in das Medium einleitet.

## Claims

1. Method for the detection of the presence of gas hydrates or of ice in a medium containing water and capable of forming solid crystals, **characterized in that** comprises at least the following stages:
- at at least one measurement point in said medium, two characteristic values of the Raman spectra corresponding to two distinct vibrational modes of the OH bonds of water are measured, said two vibrational modes corresponding to a first wavenumber located at 3160 cm⁻¹ ± 40 cm⁻¹ and to a second wavenumber located at 3400 cm⁻¹ ± 150 cm⁻¹, and the ratio τ of said two characteristic values is determined;
- the temperature T in said medium at said point of measurement of said spectra is determined;
- the ratio τ is compared with a value τ₀ corresponding to a predetermined threshold for formation of said crystals for said temperature T; and
- a presence of water in the solid form is determined from said comparison and a presence of ice or a presence of gas hydrates are distinguished between from a comparison between said measured temperature T and the temperature of the formation of the ice under the measurement conditions.

2. Method according to Claim 1, in which said characteristic value corresponds to the intensity of said spectrum for said two vibrational modes, or to a value directly related to the intensity, for example the integral of said spectrum centred on said vibrational modes.

3. Method according to either of the preceding claims, in which said temperature is varied in the vicinity of said point of measurement of said two characteristic values in order to anticipate the formation of solid crystals, for example of hydrates.

4. Method according to one of the preceding claims, in which the presence of hydrate crystals is deduced if the ratio τ is greater than a calibration value τ₀ and if the temperature measured is greater than the temperature of formation of the ice Tf under the measurement conditions.

5. Method according to one of the preceding claims, in which, in the event of presence of hydrates, a hydrate-inhibiting additive is injected into said medium.
